# EUROPÄISCHE PATENTSCHRIFT

(11) **EP 0 353 500 B1**
(45) Veröffentlichungstag und Bekanntmachung des Hinweises auf die Patenterteilung: **21.09.1994**
(21) Anmeldenummer: 89112703.7
(22) Anmeldetag: 12.07.1989
(51) Int. Cl.: G01N 33/52, G01N 33/68

(54) **Testträger zur analytischen Bestimmung eines Bestandteils einer flüssigen Probe**
Test strip for the analytical determination of a component in a fluid sample
Bande-test pour la détermination analytique d'un composant dans un échantillon liquide

(30) Priorität: 30.07.1988 DE 3826057
(43) Veröffentlichungstag der Anmeldung: 07.02.1990
(73) Patentinhaber: BOEHRINGER MANNHEIM GMBH, 68298 Mannheim (DE)
(72) Erfinder: Schlipfenbacher, Reiner, D-6840 Lampertheim (DE); Steinbiss, Joachim, Dr., D-6143 Lorsch (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 267 519
- EP-A- 0 353 501
- CLINICAL CHEMISTRY, Band 36, Nr. 6, 1990, Winston-Salem, NC (US); R.L. SCHLIPFENBACHER et al., Nr. 0219#

## Beschreibung

Die Erfindung betrifft einen Testträger zur analytischen Bestimmung eines Bestandteils einer flüssigen Probe, insbesondere für die Diagnose von Krankheiten mittels einer auf dem Testträger ablaufenden Reaktionsfolge. Der Testträger weist mehrere Testschichten auf. Hierzu gehört eine Basisschicht, auf der sich nebeneinander ein Probenaufgabebereich und ein Auswertebereich befindet. Eine Flüssigkeitstransportstrecke erstreckt sich von dem Probenaufgabebereich in den Auswertebereich. Weiter weist der Testträger eine Farbbildungsschicht auf, in der aufgrund einer Farbbildungsreaktion mit Hilfe eines Farbbildungs-Reagenzsystems, von dem mindestens ein Farbbildungsreagenz sich in der Farbbildungsschicht befindet, eine für den zu bestimmenden Bestandteil charakteristische optisch nachweisbare Veränderung stattfindet.

Für qualitative oder quantitative analytische Bestimmungen im Rahmen der Diagnose von Krankheiten werden in jüngerer Zeit zunehmend sogenannte trägergebundene Tests verwendet. Bei diesen sind Reagenzien in entsprechende Schichten eines festen Testträgers eingebettet, der mit der Probe in Kontakt gebracht wird. Die Probe ist meist eine Körperflüssigkeit wie Blut oder Urin. Es kann sich aber auch um eine durch vorausgehende Testschritte, beispielsweise durch Kontaktierung eines Eluationsmittels mit einer Stuhlprobe, gewonnene Flüssigkeit handeln.

Die Reaktion der flüssigen Probe mit den Reagenzien führt zu einem nachweisbaren Signal, wobei sich die Erfindung auf Fälle bezieht, bei denen eine für den zu bestimmenden Bestandteil charakteristische Farbänderung erzeugt wird, die in der Farbbildungsschicht stattfindet. Die Farbänderung kann visuell oder mit Hilfe eines entsprechenden Gerätes, meistens reflexionsphotometrisch, ausgewertet werden.

Es werden verschiedene Farbbildungsreaktionen verwendet, die aus mehreren Reaktionsstufen bestehen, an denen mehrere verschiedene Reagenzien beteiligt sind. Diese werden insgesamt als Farbbildungs-Reagenzsystem bezeichnet. Mindestens ein Farbbildungs-Reagenz dieses Systems befindet sich in der Farbbildungsschicht und nimmt an einer Reaktionsstufe teil, die zu der Farbänderung führt.

Testträger sind in vielen verschiedenen Gestaltungen bekannt. Häufig haben sie die Form eines Teststreifens, der auf einer länglichen Basisschicht ein oder mehrere Testfelder aufweist. Die Testfelder bestehen ihrerseits häufig aus mehreren Testschichten, die gleich groß und übereinander angeordnet sind, so daß die auf ein Testfeld aufgetragene Probenflüssigkeit senkrecht zu den Oberflächen der Testschichten durch diese hindurchsikkert. Da in diesem Fall der Flüssigkeitstransport ausschließlich quer zu den Testschichtoberflächen (und zu der Basisschicht) stattfindet, kann man solche Testträger als "Quertransport-Testträger" bezeichnen.

Die Erfindung richtet sich auf "Testträger mit Längstransport". Sie haben eine in der Regel langgestreckte Basisschicht, auf der man einen Probenaufgabebereich und einen Auswertebereich unterscheiden kann. Bei derartigen Testträgern wird die Probe im Probenaufgabebereich aufgegeben und dann längs einer Flüssigkeitstransportstrecke, die parallel zu der Basisschicht verläuft, in den Auswertebereich transportiert. Derartige Testträger haben gegenüber den Quertransport-Testträgern mit ausschließlich übereinander angeordneten Testschichten erhebliche Vorteile und sind vor allem für immunologische Analyseverfahren gut geeignet. Der Flüssigkeitstransport basiert auf Kapillarwirkung, wobei die Flüssigkeitstransportstrecke sowohl durch eine oder mehrere Testschichten aus saugfähigem Material, wie beispielsweise Papier oder Vlies, als auch durch einen Spalt, der sich durch Kapillarwirkung vollsaugt, gebildet werden kann. Testträger mit Längstransport sind beispielsweise in der DE-A 34 45 816, der DE-A 36 38 654 und der DE-A 36 43 516 beschrieben.

Wichtig für die Genauigkeit und zuverlässige Funktion von Analyse-Testträgern ist, daß sämtliche Testschichten in ausreichendem Maße mit Probenflüssigkeit benetzt werden. Andererseits soll es -um die Handhabung zu erleichtern- nach Möglichkeit nicht notwendig sein, die Probenflüssigkeitsmenge vor Aufgabe auf den Testträger genau abzumessen.

Bei den bekannten Testträgern wird üblicherweise die Probe im Überschuß aufgegeben und abgewartet, bis sich der Testträger vollständig vollgesaugt hat. Ob bzw. zu welchem Zeitpunkt dies tatsächlich geschehen ist, läßt sich meist nicht kontrollieren. Gemäß der Bedienungsanleitung soll der Benutzer einfach eine bestimmte Zeit abwarten, und danach den Probenüberschuß, beispielsweise durch Abwischen oder Abwaschen, entfernen.

In der EP-A- 256 806 ist ein Quertransport-Testträger beschrieben, bei dem die Basisschicht unterhalb des Testfeldes eine Öffnung aufweist. Die Probe wird von der anderen Seite her auf das Testfeld aufgegeben. Die Öffnung dient dazu, die Rückseite des Testfeldes reflexionsphotometrisch zu beobachten. Dadurch soll der Zeitpunkt festgestellt werden können, zu dem das Testfeld vollständig benetzt ist. Dieses Verfahren beschränkt sich jedoch auf Quertransport-Testträger. Die Ausbreitung der Flüssigkeit über die Fläche der Testschicht kann nur in dem kleinen Teilbereich, in dem sich die Öffnung befindet, kontrolliert werden und die Kontrollmöglichkeit ist auf Fälle beschränkt, bei denen die Probenflüssigkeit gefärbt ist, so daß sie unmittelbar nachgewiesen werden kann.

Um bei einem Testträger mit Längstransport der eingangs bezeichneten Art kontrollieren zu können, ob bzw. zu welchem Zeitpunkt die Testschichten des Testträgers bis zur Farbbildungsschicht hin ordnungsgemäß benetzt sind und damit die Dosierung der Probe auf dem Testträger zu verbessern, ist erfindungsgemäß in dem Auswertebereich über der von der Basisschicht abgewandten Fläche der Farbbildungsschicht eine Abdeckschicht angeordnet, die im Ausgangszustand des Testträgers so undurchsichtig ist, daß die Farbe der Farbbildungsschicht nicht sichtbar ist. Dabei steht die Abdeckschicht mit der Flüssigkeitstransportstrecke in Fluidkontakt und die Abdeckschicht und die Farbbildungsschicht sind so ausgebildet, daß die Farbe des Farbbildungsreagenz sichtbar wird, wenn beide Schichten von der Probenflüssigkeit benetzt sind.

Als Ausgangszustand wird der "trockene" Zustand des Testträgers vor Aufgabe der Probe bezeichnet.

Der Begriff "Fluidkontakt" (engl.: fluid contact) ist eine in der Testträgertechnik üblich gewordene Bezeichnung dafür, daß zwei Testschichten so zueinander angeordnet sind, daß zwischen ihnen ein Flüssigkeitsaustausch, meist aufgrund der Saugwirkung der Testschichten, möglich ist. Die Testschichten können dabei in unmittelbarem Kontakt zueinander stehen oder der Fluidkontakt kann indirekt, z.B. über dazwischenliegende saugfähige Schichten bewirkt werden.

Die Abdeckschicht besteht aus einem saugfähigen Material. Geeignet ist beispielsweise Papier, ein textiles Material (Gewebe, Vlies) oder eine poröse Kunststoffschicht.

Jedes derartige Material hat Spalten oder Poren, in denen die Flüssigkeit durch Kapillarkräfte transportiert wird.

Zwischen der Abdeckschicht und der Farbbildungsschicht sollte ein flächiger planer Kontakt bestehen. Beide Schichten können miteinander verbunden sein, soweit dadurch der Flüssigkeitsaustausch nicht in störendem Maße beeinflußt wird. Bevorzugt liegen sie jedoch lose aufeinander auf und werden in geeigneter Weise, beispielsweise durch eine Niederhalterschicht, gegeneinander gedrückt.

Wesentlich für die vorliegende Erfindung ist das Zusammenwirken des Längstransportes der Flüssigkeit und der Sichtbarmachung des Zeitpunktes, zu dem der Längstransport abgeschlossen ist. Wenn man den Testträger im Probenaufgabebereich mit der Probe in Kontakt bringt, strömt diese den Testträger entlang bis zu dem Nachweisbereich. Dieser Vorgang ist weitgehend unabhängig von der aufgegebenen Probenmenge, solange sie nur zur vollständigen Benetzung aller Testschichten ausreicht. Erfindungsgemäß wird nun der Zeitpunkt der vollständigen Füllung des Testträgers signalisiert. Dadurch ist nicht nur eine Kontrolle der einwandfreien Funktion möglich, sondern es wird vielfach auch eine Verbesserung der Reproduzierbarkeit der Flüssigkeitsdosierung erreicht.

Damit die Farbe der Farbbildungsschicht sichtbar wird, wenn die Abdeckschicht und die Farbbildungsschicht mit Probenflüssigkeit benetzt sind, ist es vorteilhaft, die Farbbildungsschicht so auszubilden, daß das Farbbildungsreagenz bei Benetzung mit der Flüssigkeit in dieser gelöst oder dispergiert und dadurch in den flüssigen Zustand überführt wird. Das Farbbildungsreagenz kann dadurch in die Abdeckschicht eindringen, so daß seine Farbe sichtbar wird. Die Farbbildungsreaktion findet in diesem Fall bevorzugt überwiegend in der Abdeckschicht statt, so daß die Änderung der Farbe gut beobachtet oder reflexionsphotometrisch gemessen werden kann.

Eine Farbbildungsschicht mit einem gut löslichen Farbbildungsreagenz kann durch Imprägnieren einer Trägermatrix hergestellt werden. Da die Probenflüssigkeit sich zunächst in der Abdeckschicht lateral (parallel zur Schichtoberfläche) ausbreitet, kann sich dabei allerdings das Problem ergeben, daß sich ein schnell lösliches farbbildendes Reagenz in der fortschreitenden Flüssigkeitsfront anreichert und dadurch eine inhomogene Farbbildung verursacht wird.

Um dies zu vermeiden, sollte die Flüssigkeitstransportgeschwindigkeit in der Abdeckschicht und der zeitliche Beginn der Farbbildungsreaktion in der Farbbildungsschicht derartig aufeinander abgestimmt sein, daß sich zunächst die Flüssigkeit in der Abdeckschicht ausbreitet und die Farbbildungsreaktion im wesentlichen danach stattfindet.

Vorzugsweise ist aus diesem Grunde die Farbbildungsschicht so ausgebildet, daß das Farbbildungsreagenz verzögert freigesetzt wird. Dies kann durch eine verzögerte Löslichkeit des Farbbildungsreagenz selbst bewirkt werden. Bevorzugt wird die verzögerte Freisetzung jedoch dadurch erreicht, daß das Farbbildungsreagenz in einer verzögert löslichen Filmschicht eingebettet ist, die sich auf einem Träger in Form einer Folie oder eines Gewebes befindet.

Gemäß einer weiteren besonders bevorzugten Ausführungsform ist dabei das Farbbildungsreagenz selbst schneller löslich, als die Filmschicht, in die es eingebettet ist. Dadurch wird erreicht, daß das Farbbildungsreagenz, wenn es aus der Signalbildungsschicht freigesetzt ist, in flüssiger Phase schnell und homogen reagiert.

Es ist offensichtlich, daß trotz der Verzögerung der Farbbildungsreaktion diese in geringem Umfange schon einsetzt, während sich die Flüssigkeit noch in der Abdeckschicht ausbreitet. Es ist jedoch ausreichend, wenn die Farbbildungsreaktion im dem Sinne "im wesentlichen" nach der Flüssigkeitsausbreitung stattfindet, daß eine auf der ganzen Signalbildungsschicht homogene Signalbildung erreicht wird.

Um das gewünschte verzögerte Lösungsverhalten zu bewirken, enthält die Farbbildungsschicht bevorzugt quellbare Makromoleküle und/ oder einen hydrophoben Filmbildner.

Quellbare Makromoleküle in diesem Sinne werden auch als Hydrokolloide bezeichnet. Es sind makromolekulare hydrophile Substanzen, die in Wasser löslich oder zumindest dispergier- und quellbar sind. Hierzu gehören insbesondere Polysaccharide wie z.B. Exudate (z.B. Gummi arabicum), Samenmehle (z.B. Johannisbrotkernmehl, Stärke), Extrakte aus Pflanzen (z.B. Pektine, Alginate), mikrobielle Polysaccharide (z.B. Xantangummi) und chemisch modifizierte Polysaccharide (z.B. Cellulose-Derivate).

Auch bestimmte Proteine sind zu dieser Substanzgruppe zuzurechnen, wie z.B. Skleroproteine und ihre Hydrolysate (z.B. Collagen, Crotein C), schwer lösliche Reserveproteine (z.B. Zein, gefälltes Casein), und hydrophob auftrocknende Proteine in entsprechend hohen Konzentrationen (z.B. Serumalbumin).

Als hydrophobe Filmbildner im Sinne der Erfindung werden wasserlösliche Polymere verstanden, die bei der Trocknung aus einer wässrigen Lösung einen verzögert anlösbaren Film bilden, in dem durch die Klebewirkung des Polymeren die Filminhaltsstoffe verfestigt und gebunden werden.

Die Auflösungseigenschaften solcher Filme können in weitem Maß durch Auswahl der Polymeren gesteuert werden. Hierzu gehören beispielsweise Polyvinylalkohole, wie sie unter dem Markenzeichen "Mowiol" von der Hoechst AG, Frankfurt, Bundesrepublik Deutschland (BRD), vertrieben werden, Polyäthylenoxyd, wie es unter dem Markennamen "Polyox" von der Union Carbide Corp., New York, USA, vertrieben wird, oder Acrylharze, wie sie von der Firma Roehm, Darmstadt, BRD unter dem Markenzeichen "Eudragid" erhältlich sind.

Eine zweite bevorzugte Maßnahme, um die Farbe des Farbbildungsreagenz durch die Abdeckschicht hindurch sichtbar werden zu lassen, besteht darin, die Abdeckschicht aus einem Material herzustellen, das in trockenem Zustand opak, im feuchten Zustand jedoch transparent ist. Der Begriff "opak" ist hierbei so zu verstehen, daß die Schicht so weitgehend undurchsichtig ist, daß die Farbe der Farbbildungsschicht nicht oder nur sehr schwach zu erkennen ist. Entscheidend ist, daß ein deutlicher Unterschied in der Durchsichtigkeit zwischen dem trockenen und dem feuchten Zustand festzustellen ist.

Als besonders gut geeignetes Material für die Abdeckschicht hat sich eine Kunststoffmembran, also eine feinporige Kunststoffschicht erwiesen. Einen deutlichen Unterschied bezüglich der Durchsichtigkeit im feuchten und trockenen Zustand zeigt hydrophiles Polyvinylidendifluorid, wie es in dem US-Patent 4 618 533 beschrieben wird. Aufgrund der vorliegenden Beschreibung kann der Fachmann jedoch auch andere Materialien und insbesondere Kunststoffmembranen auswählen, die die genannten Bedingungen erfüllen. Dabei dürfte der Brechungsindex des Kunststoffs eine wesentliche Rolle spielen. Es ist anzunehmen, daß Materialien, deren Brechungsindex nahe bei dem der Probenflüssigkeit liegt, die Eigenschaft haben, im feuchten Zustand transparent zu werden.

Besonders vorteilhaft ist es, wenn eine im feuchten Zustand transparente Abdeckschicht in Verbindung mit einem löslichen, aus der Farbbildungsschicht in die Abdeckschicht übertretenden Farbbildungsreagenz eingesetzt wird.

Die Erfindung eignet sich vor allem für einen Testträger zur Durchführung immunologischer Bestimmungen, wie im folgenden anhand eines in den Figuren schematisch dargestellten Ausführungsbeispiels näher erläutert wird. Es zeigen:
- Fig. 1: eine perspektivische Darstellung eines erfindungsgemäßen Testträgers,
- Fig. 2: eine Detailansicht aus Fig. 1.

Der in den Figuren dargestellte Testträger 1 hat eine Basisschicht 2, auf der die übrigen Testschichten befestigt sind. In seiner Längsrichtung läßt sich der Testträger in einen Probenaufgabebereich 4 und in einen Auswertebereich 6 unterteilen. In dem Probenaufgabebereich 4 sind nebeneinander eine Konjugatschicht 8 und eine Flüssigkeitstransportschicht 9 auf der Basisschicht 2 mit Hilfe von Schmelzkleber 10 befestigt. Die Schicht 8 überlappt geringfügig die anschließende Schicht 9 um einen möglichst guten Fluidkontakt zwischen ihnen zu gewährleisten. Die Schichten 8 und 9 bilden eine Flüssigkeitstransportstrecke, die sich von dem Probenaufgabe- und Vorreaktionsbereich 4 in den Auswertebereich 6 erstreckt.

Im dargestellten Beispielsfall wird die Probe auf die Konjugatschicht 8 aufgegeben, wobei diese Schicht gleichzeitig dazu dient, eine erste Reaktionsstufe durchzuführen. Der Probenaufgabebereich 4 dient zugleich als Vorreaktionsbereich.

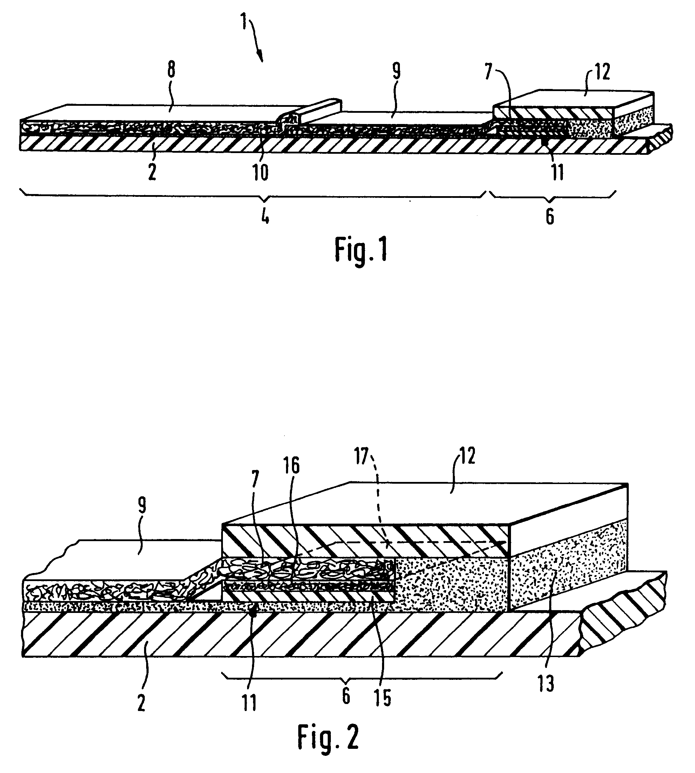

In dem Auswertebereich 6 sind auf der Basisschicht 2, wie in Fig. 2 deutlicher zu erkennen ist, übereinander eine Farbbildungsschicht 11, eine Abdeckschicht 7 und eine Niederhalterschicht 12 zu erkennen. Die Niederhalterschicht 12 besteht aus einer verhältnismäßig steifen Kunststoffolie. Sie ist mit Hilfe eines Schmelzkleberstreifens 13 entsprechend großer Schichtdicke an der Basisschicht 2 so befestigt, daß sie parallel zu ihr mit einem Abstand verläuft, der etwa der Gesamtdicke von Farbbildungsschicht 11 und Abdeckschicht 7 entspricht. Die Niederhalterschicht 12 hat eine ausreichende Steifigkeit, um die zwischen ihr und der Basisschicht 2 befindlichen Schichten so zusammenzudrücken, daß ein guter Fluidkontakt zwischen ihnen gewährleistet ist.

Bei der dargestellten bevorzugten Ausführungsform sind neben der Farbbildungsschicht auf ihrer in Längsrichtung der Basisschicht 2 von dem Probenaufgabebereich 4 abgewandten Seite (also in Fig. 1 rechts von der Farbbildungsschicht 11) keine weiteren saugfähigen Schichten vorgesehen. Die Farbbildungsschicht 11 steht also mit dem in Flüssigkeitstransportrichtung letzten Teilstück der Flüssigkeitstransportstrecke 8,9,7 in Fluidkontakt.

Die Farbbildungsschicht 11 besteht in der dargestellten bevorzugten Ausführungsform aus einer Tragfolie 15 und einer darauf befindlichen verzögert löslichen Filmschicht 16, die ein Farbbildungsreagenz enthält.

Der in den Figuren dargestellte Testträger ist, wie erwähnt, insbesondere für immunologische Bestimmungen geeignet. Derartige Bestimmungen benutzen hochspezifische Bindungsreaktionen zwischen verschiedenen Spezies, die man als Bindungspartner bezeichnen kann. Immunologische Bindungspartner sind insbesondere Antikörper einerseits, sowie Antigene oder Haptene andererseits.

Für den Fall, daß ein in der Probe enthaltenes Antigen AG als Analyt bestimmt werden soll, ist beispielsweise folgender Testablauf typisch.

Die Probe wird auf die Konjugatschicht 8 aufgegeben. Die Konjugatschicht enthält ein lösliches Konjugat AKE eines mit dem AG spezifisch bindungsfähigen Antikörpers AK mit einem Enzym E. Durch die spezifische Bindungsreaktion entstehen Komplexe AG-AKE.

Überschüssiges AKE gelangt zusammen mit den AG-AKE-Komplexen in die Flüssigkeitstransportschicht 9. Diese enthält ein Antigen AGF in trägerfixierter Form. Das AGF ist mit dem Probenantigen identisch oder analog zu diesem, d.h. mit dem Antikörper des in der Konjugatschicht 8 enthaltenden AKE spezifisch bindungsfähig.

Das überschüssige freie AKE wird nun aufgrund der spezifischen Bindungsreaktion mit dem in der Schicht 9 fixierten Antigen trägerfixiert. Für die Funktion ist es wichtig, daß die Belegungsdichte des fixierten Antigens auf der Schicht 9 hoch genug ist, daß praktisch das gesamte überschüssige Konjugat daran gebunden wird. Die Schicht 9 kann deshalb auch als "Fixierungsschicht" bezeichnet werden. Nur die freien AG-AKE-Komplexe gelangen in die Auswertezone 6. Dadurch entspricht die Menge der in der Auswertezone 6 eintreffenden AG-AKE-Komplexe (und damit die Menge des Markierungsenzyms) der Menge des Analyten.

Die Probenflüssigkeit mit den AG-AKE-Komplexen strömt weiter in die Abdeckschicht 7 und füllt diese vollständig, im wesentlichen bevor die Farbbildungsreaktion mit dem Farbbildungsreagenz in der Schicht 11 beginnt. Der verzögerte Beginn der Farbbildungsreaktion wird, wie oben dargelegt, insbesondere dadurch erreicht, daß sich die Schicht 11 verzögert löst.

Die Abdeckschicht 7 ist, wie in Fig. 2 besonders deutlich zu erkennen ist, mit der Fixierungsschicht 9 einstückig hergestellt, d.h. beide Schichten bestehen aus einem Streifen des gleichen Schichtmaterials. Dies ist bevorzugt, jedoch nicht notwendig. Die Abdeckschicht 7 könnte auch eine separate Schicht sein, die in irgendeiner Weise mit der Flüssigkeitstransportstrecke 8,9 in Fluidkontakt steht.

Die Flüssigkeit dringt senkrecht zu den Schichtoberflächen gleichmäßig durch die in Fig. 2 gestrichelt dargestellte Flüssigkeitsaustauschfläche 17 in die Farbbildungsschicht 11 ein. Die Farbbildungsschicht 11 enthält ein Substrat für das Enzym E. In Abhängigkeit von der Enzymkonzentration findet ein Farbumschlag statt, der ein Maß für die Konzentration des Analyten ist.

Die visuelle oder apparative Auswertung des Farbumschlags erfolgt von der Seite der Abdeckschicht her. Solange diese trocken ist, verdeckt sie die Farbe der Farbbildungsschicht. Nachdem sich die Abdeckschicht mit Flüssigkeit gefüllt hat und die Farbbildungsschicht zumindest oberflächlich benetzt ist, wird dagegen deren Farbe sichtbar. Dies kann, wie oben erläutert, dadurch erreicht werden, daß das Farbreagenz aus der Farbbildungsschicht 11 in die flüssige Phase übertritt und weiter in die Abdeckschicht 7 gelangt. Zusätzlich oder alternativ kann die Abdeckschicht aus einem im flüssigen Zustand durchscheinend werdenden Material bestehen.

In einem praktischen Beispiel ist das Farbreagenz im Ausgangszustand gelb gefärbt. Die Abdeckschicht ist undurchsichtig weiß, so daß der Auswertebereich für den Betrachter von der Auswerteseite her weiß erscheint. Sobald die Abdeckschicht mit der flüssigen Probe benetzt ist, wird die gelbe Farbe sichtbar. Daran kann der Beobachter erkennen, daß ausreichend Probe aufgebracht wurde und der Längstransport der Probenflüssigkeit in dem Testträger einwandfrei stattgefunden hat. Diese farbliche Änderung kann auch apparativ nachgewiesen werden. Dadurch ist eine vollautomatische Kontrolle der richtigen Funktion des Teststreifens möglich. Die farbliche Änderung kann dazu benutzt werden, einen Meßvorgang einzuleiten.

Diese Kontrollfunktion ist unabhängig von der Färbung der Probenflüssigkeit. Dies ist besonders wichtig, wenn weitgehend farblose Flüssigkeiten, wie Urin oder Plasma analysiert werden sollen.

Als Material für die Abdeckschicht und gegebenenfalls auch für die einstückig aus dem gleichen Material hergestellte Fixierungsschicht 9 sind insbesondere dünne Kunststoffmembranen geeignet. Die Materialstärke soll bevorzugt unter 1 mm, besonders bevorzugt unter 0,3 mm liegen. Geeignet sind beispielsweise folgende Materialien
Biodyne BNRG der Fa. Pall, Glencove, N.Y., USA 150µm
SM 119 der Fa. Sartorius, Göttingen, BRD 140µm
Nitrocellulose der Fa. BioRad, Richmond, Ca, USA 130µm
NC der Fa. Schleicher und Schüll, Dassel, BRD 150µm

Besonders vorteilhaft sind folgende Materialien, die im trockenen Zustand lichtundurchlässig weiß und im feuchten Zustand transparent sind:
Durapore und Immobilon , beide hergestellt von der Fa. Millipore, Bedford, USA.

Die Funktionsweise des Testträgers wurde beispielhaft für den Fall beschrieben, daß ein Antigen bestimmt werden soll. Ein analoger Testverlauf ist auch zur Bestimmung eines Antikörpers möglich, wobei dann ein Antigenkonjugat in der Schicht 8 und ein trägerfixierter analoger Antikörper in der Schicht 9 eingesetzt werden müßte.

Allgemein ist der erfindungsgemäße Testträger besonders für solche Bestimmungen geeignet, bei denen die Reaktionsfolge eine spezifische Bindungsreaktion zwischen einem mit der Konzentration des zu bestimmenden Bestandteils korrelierten ersten Bindungspartner (im Beispielsfall AG) und einem markierten zweiten Bindungspartner (im Beispielsfall AKE) unter Bildung beweglicher Komplexe (im Beispielsfall AG-AKE) und eine weitere spezifische Bindungsreaktion zwischen dem zweiten Bindungspartner (im Beispielsfall wiederum AKE) und einem zum ersten Bindungspartner analogen trägerfixierten dritten Bindungspartner (im Beispielsfall AGF) umfaßt. Dabei ist notwendig, daß der zweite und der dritte Bindungspartner jeweils bezogen auf die Strömungsrichtung der Flüssigkeit in dem Testträger vor dessen Nachweisbereich angeordnet sind. Dadurch ist die spezifische Bindungsreaktion abgeschlossen, bevor die für die Analyse spezifischen freien Komplexe in den Nachweisbereich gelangen.

Der beschriebene immunologische Testablauf ist -abgesehen von den Besonderheiten der jeweiligen Erfindung- ähnlich dem in der DE-A 36 38 654 beschrieben. Auf diese Publikation wird daher ergänzend Bezug genommen.

Der erfindungsgemäße Testträger ist als Nachweiseinheit für einen Testkit zur Bestimmung eines Analyten im Stuhl besonders geeignet, wie er in EP-A-291 843 beschrieben wird. Dieser Testkit hat eine Probensammeleinheit, in der aus den Stuhlproben durch Eluation mit Hilfe eines Eluationsmittels eine Flüssigkeit gewonnen wird, die den Analyten enthält. Die so gewonnene Probenflüssigkeit kann vorteilhaft mit dem Testträger gemäß der vorliegenden Erfindung untersucht werden.

Das folgende Beispiel 1 betrifft einen solchen Testträger, bei dem als Analyt human Serum Albumin (hSA) bestimmt wird, das aus einer Stuhlprobe eluiert wurde und ein Indikator für die Anwesenheit von Blut im Stuhl ist.

### Beispiel 1:

Ein Testträger gemäß den Figuren 1 und 2 wird wie folgt hergestellt:
a) Konjugatschicht 8:
   IgG <human Serum Albumin> wird mit β-Galaktosidase kovalent konjugiert. Dieses Konjugat wird in ein Glasfaservlies eingetränkt und getrocknet. Die Testschichtgröße auf dem Testträger beträgt 20 x 6 mm.
b) Fixierungsschicht 9 und Abdeckschicht 7:
   An eine Membran aus hydrophilem Polyvinylidendifluorid (PVDF) der Firma Millipore (Bedford, USA) die unter dem Markennamen Immobilon AV vertrieben wird, wird hSA kovalent fixiert. Die Flächenkonzentration wird über die Konzentration in dem für den Tränkungsvorgang verwendeten Puffer auf 20 µg hSA/cm² eingestellt. Die Schichtgröße beträgt 20 x 6 mm.
c) Signalbildungsschicht 11:
   Eine filmbildende Beschichtungsmasse wird hergestellt auf Basis von 0,6 % Ketrol F der Fa. Kelco, Hamburg, Bundesrepublik Deutschland (BRD) unter Zusatz von 2,5 % Methylcellulose 15 der Fa. Serva, Heidelberg, BRD. Sie enthält 12 mM ChlorPhenolRot-β-Galaktosid (CPRG) und ist in HEPES gepuffert. Die Beschichtungsmasse wird in einer Filmschichtstärke von 200 µm auf eine 100 µm starke Tragfolie aus Pokalon der Fa. Lonza, Weil/Rh., BRD beschichtet. Die Schichtgröße beträgt 6 x 6 mm.
d) Niederhalterschicht 12:
   Diese besteht aus einer 140 µm starken Pokalonfolie.

Als Basisschicht wird eine Polyesterfolie "Melinex" der Firma ICI, Frankfurt, BRD verwendet. Die Verklebung der Komponenten erfolgt mit Schmelzkleber Dynapol S 1358 der Fa. Dynamid Nobel, Troisdorf, BRD.

Die Flüssigkeitsfront bewegt sich in der Schicht 9,7 verhältnismäßig langsam, so daß bis zur vollständigen Ausbreitung in der Schicht 7 nahezu 3 min. benötigt werden. Durch das verzögerte Lösungsverhalten der Substratschicht 11 wird dennoch eine völlig homogene Farbbildung in Abhängigkeit von der hSA-Konzentration erreicht, die eine Bestimmung dieser Konzentration mit guter Genauigkeit erlaubt.

Die Abdeckschicht des Testträgers ist zunächst weiß. Erst wenn die Flüssigkeitsfront die Abdeckschicht erreicht hat, wozu ca. 180 sec. benötigt werden, erfolgt ein Farbumschlag nach gelb, der dem Benutzer anzeigt, daß sich die Probenflüssigkeit vollständig in dem Testträger ausgebreitet hat. Nun kann er durch die Abdeckschicht hindurch den Ablauf der Farbreaktion beobachten. Im Beispielsfall erfolgt innerhalb von etwa weiteren 180 sec. ein Farbumschlag nach rot, wenn eine über dem Schwellwert liegende Menge an human Serum Albumin in der Probe enthalten war.

## Patentansprüche

1. Testträger zur analytischen Bestimmung eines Bestandteils einer flüssigen Probe, insbesondere für die Diagnose von Krankheiten, mittels einer auf dem Testträger ablaufenden Reaktionsfolge,
mit mehreren Testschichten, die im Ausgangszustand trocken sind und bei der Benutzung von der Probenflüssigkeit benetzt werden, umfassend
eine Basisschicht (2), auf der sich nebeneinander ein Probenaufgabebereich (4) und ein Auswertebereich (6) befinden, eine Flüssigkeitstransportstrecke, die sich von dem Probenaufgabebereich (4) in den Auswertebereich (6) erstreckt, und eine Farbbildungsschicht (11) in der aufgrund einer Farbbildungsreaktion mit Hilfe eines Farbbildungs-Reagenzsystems, von dem mindestens ein Farbbildungsreagenz sich in der Farbbildungsschicht (11) befindet, eine für den zu bestimmenden Bestandteil charakteristische optisch nachweisbare Veränderung stattfindet,
**dadurch gekennzeichnet**, daß
in dem Auswertebereich (6) über der von der Basisschicht (2) abgewandten Fläche der Farbbildungsschicht (11) eine saugfähige Abdeckschicht (7) angeordnet ist, die im trockenen Ausgangszustand des Testträgers vor Aufgabe der Probe so undurchsichtig ist, daß die Farbe der Farbbildungsschicht nicht sichtbar ist,
die Abdeckschicht (7) mit der Flüssigkeitstransportstrecke in Fluidkontakt steht, und
die Abdeckschicht (7) und die Farbbildungsschicht (11) so ausgebildet sind, daß die Farbe des Farbbildungsreagenz sichtbar wird, wenn beide Schichten von der Probenflüssigkeit benetzt sind.

2. Testträger nach Anspruch 1, **dadurch gekennzeichnet**, daß die Farbbildungsschicht (11) so ausgebildet ist, daß das Farbbildungsreagenz bei Benetzung mit der Flüssigkeit in die flüssige Phase überführt wird, so daß es in die Abdeckschicht (7) eindringt und die Farbbildungsreaktion überwiegend in der Abdeckschicht stattfindet.

3. Testträger nach Anspruch 1 oder 2, **dadurch gekennzeichnet**, daß die Flüssigkeitstransportgeschwindigkeit in der Abdeckschicht (7) und der zeitliche Beginn der Farbbildungsreaktion in der Farbbildungsschicht (11) derart aufeinander abgestimmt sind, daß sich zunächst die Flüssigkeit in der Abdeckschicht (7) ausbreitet und die Farbbildungsreaktion im wesentlichen danach stattfindet.

4. Testträger nach einem der Ansprüche 1 bis 3, **dadurch gekennzeichnet**, daß die Abdeckschicht (7) aus einem im trockenen Zustand opaken, im feuchten Zustand jedoch transparenten Material besteht.

5. Testträger nach einem der Ansprüche 1 bis 4, **dadurch gekennzeichnet**, daß die Abdeckschicht (7) ein Kunststoffmembran aufweist.

6. Testträger nach Anspruch 5, **dadurch gekennzeichnet**, daß die Kunststoffmembran aus Polyvinyliden-di-fluorid besteht.

7. Testträger nach Anspruch 1, **dadurch gekennzeichnet**, daß die Abdeckschicht (7) weniger als 1 mm, bevorzugt weniger als 0,3 mm dick ist.

8. Testträger nach Anspruch 1, **dadurch gekennzeichnet**, daß die Flüssigkeitstransportstrecke eine Flüssigkeitstransportschicht (9) aufweist, die sich einstückig aus dem Probenaufgabebereich (4) bis über die gesamte Farbbildungsschicht (11) erstreckt und zugleich die Abdeckschicht (7) bildet.

9. Testträger nach Anspruch 1, **dadurch gekennzeichnet,** daß keine weiteren saugfähigen Testschichten neben der Farbbildungsschicht (11) und der Abdeckschicht (7) auf deren in Längsrichtung der Basisschicht von dem Probenaufgabebereich (4) abgewandten Seite vorgesehen sind.

10. Testträger nach Anspruch 1, **dadurch gekennzeichnet,** daß eine Niederhalterschicht (12) an der Basisschicht befestigt ist und die Signalbildungsschicht (11) und die Abdeckschicht (7) im Bereich der Flüssigkeitsaustauschfläche (17) zwischen der Basisschicht (2) und der Niederhalterschicht (12) so angeordnet sind, daß sie zusammengedrückt werden.

11. Testträger nach Anspruch 1, **dadurch gekennzeichnet**, daß die Reaktionsfolge eine spezifische Bindungsreaktion zwischen einem mit der Konzentration des zu bestimmenden Bestandteils korrelierten ersten Bindungspartner und einem markierten zweiten Bindungspartner unter Bildung beweglicher Komplexe und eine spezifische Bindungsreaktion zwischen dem zweiten Bindungspartner und einem zu dem ersten Bindungspartner analogen trägerfixierten dritten Bindungspartner unter Bildung fixierter Komplexe umfaßt, und
daß die Farbbildungsreaktion eine Reaktion zwischen dem Farbbildungsreagenz und den freien Komplexen einschließt.

## Claims

1. Test carrier for the analytical determination of a component of a liquid sample, especially for the diagnosis of diseases, by means of a reaction sequence taking place on the test carrier with several test layers which, in the initial state, are dry and in use are wetted by the sample liquid comprising
a base layer (2) on which are present side by side a sample application region (4) and an evaluation region (6), a liquid transport path which extends from the sample application region (4) into the evaluation region (6) and a colour-formation layer (11) in which, on the basis of a colour-formation reaction with the help of a colour-formation reagent system, at least one colour-formation reagent of which is present in the colour-formation layer (11), there takes place an optically detectable change characteristic for the component to be determined,
characterized in that
in the evaluation region (6) above the surface of the colour-formation layer (11) facing away from the base layer (2), there is arranged an absorbent covering layer (7) which, in the initial state of the test carrier, is so non-transparent that the colour of the colour-formation layer is not visible,
the covering layer (7) is in fluid contact with the liquid transport path and
the covering layer (7) and the colour-formation layer (11) are designed in such a manner that the colour of the colour-formation reagent becomes visible when both layers are wetted by the sample liquid.

2. Test carrier according to claim 1, characterized in that the colour-formation layer (11) is made in such a manner that the colour-formation reagent, in the case of wetting with the liquid, is transferred into the liquid phase so that it penetrates into the covering layer (7) and the colour-formation reaction takes place preponderantly in the covering layer.

3. Test carrier according to claim 1 or 2, characterized in that the rate of liquid transport in the covering layer (7) and the starting time of the colour-formation reaction in the colour-formation layer (11) are adapted to one another in such a manner that the liquid first spreads out in the covering layer (7) and the colour-formation reaction takes place essentially thereafter.

4. Test carrier according to one of claims 1 to 3, characterized in that the covering layer (7) consists of a material which is opaque in a dry state but is transparent in a moist state.

5. Test carrier according to one of claims 1 to 4, characterized in that the covering layer (7) comprises a synthetic material membrane.

6. Test carrier according to claim 5, characterized in that the synthetic material membrane consists of polyvinylidene difluoride.

7. Test carrier according to claim 1, characterized in that the covering layer (7) is less than 1 mm, preferably less than 0.3 mm thick.

8. Test carrier according to claim 1, characterized in that the liquid transport path comprises a liquid transport layer (9) which extends in one piece from the sample application region (4) to over the whole colour-formation layer (11) whereby it simultaneously forms the covering layer (7).

9. Test carrier according to claim 1, characterized in that no further absorbent test layers are provided beside the colour-formation layer (11) and the covering layer (7) on its side facing away in the longitudinal direction of the base layer from the sample application region (4).

10. Test carrier according to claim 1, characterized in that a holding-down layer (12) is fixed on the base layer (2) and the colour-formation layer (11) and the covering layer (7) are arranged in the region of the liquid exchange surface (17) between the base layer (2) and the holding-down layer (12) in such a manner that they are pressed together.

11. Test carrier according to claim 1, characterized in that the reaction sequence comprises a specific binding reaction between a first binding partner correlated with the concentration of the component to be determined and a labelled second binding partner resulting in the formation of mobile complexes, and a specific binding reaction between the second binding partner and a carrier-bound third binding partner analogous to the first binding partner resulting in the formation of fixed complexes, and
the colour-formation reaction includes a reaction between the colour-formation reagent and the free complexes.

## Revendications

1. Bande de test pour la détermination analytique d'un constituant d'un échantillon liquide, en particulier pour le diagnostic de maladies, au moyen d'une suite de réactions se déroulant sur le support de test, celui-ci comportant plusieurs couches de test qui sont sèches à l'état initial et qui sont mouillées par l'échantillon liquide, le support de test comprenant
une couche de base (2), sur laquelle se trouvent une zone d'application de l'échantillon (4) et une zone d'évaluation (6), une voie de transport de liquide qui s'étend depuis la zone d'application de l'échantillon (4) jusqu'à la zone d'évaluation (6), et
une couche chromogène (11) dans laquelle se produit une variation optiquement détectable, caractéristique du constituant à déterminer, réalisable au moyen d'une réaction chromogène à l'aide d'un système de réactifs chromogènes, parmi lesquels au moins un réactif chromogène se trouve dans la couche chromogène (11),
caractérisé en ce que dans la zone d'évaluation (6), sur la face de la couche chromogène (11) opposée à la couche de base (2), se trouve une couche de recouvrement (7) absorbante qui, dans l'état initial du support de test, avant l'application de l'échantillon, est si peu transparente que la couleur de la couche chromogène n'est pas visible, la couche de recouvrement (7) est en contact de fluide avec la voie de transport de liquide, et la couche de recouvrement (7) et la couche chromogène (11) sont conçues de telle façon que la couleur du réactif chromogène devient visible lorsque les deux couches sont mouillées par l'échantillon liquide.

2. Support de test selon la revendication 1, caractérisé en ce que la couche chromogène (11) est conçue de telle façon que le réactif chromogène est transféré dans la phase liquide lorsque ladite couche est mouillée par le liquide, de sorte que ledit réactif imprègne la couche de recouvrement (7) et que la réaction chromogène se déroule de façon prépondérante dans la couche de recouvrement.

3. Support de test selon la revendication I ou 2, caractérisé en ce que la vitesse de transport du liquide dans la couche de recouvrement (7) et le début de la réaction chromogène dans la couche chromogène (11) sont ajustés réciproquement de façon que le liquide se répande d'abord dans la couche de recouvrement (7) et que la réaction chromogène se déroule essentiellement après.

4. Support de test selon l'une quelconque des revendications l à 3, caractérisé en ce la couche de recouvrement (7) est constituée d'un matériau qui est opaque à l'état sec, mais transparent à l'état humide.

5. Support de test selon l'une quelconque des revendications l à 4, caractérisé en ce la couche de recouvrement (7) comporte une membrane en matière plastique.

6. Support de test selon la revendication 5, caractérisé en ce que la membrane en matière plastique est en poly(difluorure de vinylidène).

7. Support de test selon la revendication 1, caractérisé en ce que la couche de recouvrement (7) présente une épaisseur inférieure à 1 mm, de préférence inférieure à 0,3 mm.

8. Support de test selon la revendication 1, caractérisé en ce que la voie de transport de liquide comprend une couche de transport de liquide (9) qui s'étend d'un seul tenant depuis la zone d'application de l'échantillon (4) sur toute la couche chromogène (11) et constitue également la couche de recouvrement (7).

9. Support de test selon la revendication 1, caractérisé en ce qu'il n'est pas prévu d'autres couches de test absorbantes, en plus de la couche chromogène (11) et de la couche de recouvrement (7), sur leur côté opposé à la zone d'application de l'échantillon (4) dans le sens longitudinal de la couche de base.

10. Support de test selon la revendication 1, caractérisé en ce qu'une couche de pression (12) est fixée sur la couche de base et que la couche de formation de signal (11) et la couche de recouvrement (7) sont disposées, dans la zone de la surface d'échange de liquide (17), entre la couche de base (2) et la couche de pression (12), de façon à être pressées l'une contre l'autre.

11. Support de test selon la revendication 1, caractérisé en ce que la suite des réactions comprend une réaction de liaison spécifique entre un premier partenaire de liaison en corrélation avec la concentration du constituant à déterminer et un second partenaire de liaison marqué, avec formation de complexes mobiles, et une réaction de liaison spécifique entre le second partenaire de réaction et un troisième partenaire de réaction fixé sur un support, analogue au premier partenaire de réaction, en formant des complexes fixés, et que la réaction chromogène comprend une réaction entre le réactif chromogène et les complexes libres.
